# EUROPEAN PATENT SPECIFICATION

(11) **EP 2 225 202 B1**
(45) Date of publication and mention of the grant of the patent: **28.12.2011**
(21) Application number: 08855641.0
(22) Date of filing: 19.11.2008
(51) Int. Cl.: C07D 209/44, C07D 471/04

(54) **Preparation of dihydropyrrol derivatives as intermediates**
Verfahren zur Herstellung von Dihydropyrrolderivaten als Zwischenprodukte
Préparation de dérivés dihydropyrroles en tant qu' intermédiaires

(30) Priority: 29.11.2007 EP 07121841
(43) Date of publication of application: 08.09.2010
(73) Proprietor: F. Hoffmann-La Roche AG, 4070 Basel (CH)
(72) Inventor: PFLEGER, Christophe, 68200 Mulhouse (FR); SPURR, Paul, CH-4125 Riehen (CH); TRUSSARDI, René, CH-4127 Birsfelden (CH); WALDMEIER, Pius, CH-4317 Wegenstetten (CH); WANG, Shaoning, CH-4058 Basel (CH)
(74) Representative: Poppe, Regina
(86) International application number: PCT/EP2008/065820
(87) International publication number: WO 2009/068463

(56) References cited:
- EP-A- 0 275 064
- WO-A-2006/082001
- YOU-XIONG WANG ET AL.: "1-methyl-3-pyrrolines and 2-methylisoindolines: new classes of cyclic tertiary amine monamine oxidase B substrates" BIOORGANIC & MEDICINAL CHEMISTRY., vol. 6, 1998, pages 143-149, XP002533970 GBELSEVIER SCIENCE LTD.
- DATABASE BEILSTEIN BEILSTEIN INSTITUTE FOR ORGANIC CHEMISTRY, FRANKFURT-MAIN, DE; Database-accession no. 5256592 (REACTION ID) XP002533971 & BESSARD, YVES ET AL.: HETEROCYCLES, vol. 51, no. 11, 1999, pages 2589-2602, ELSEVIER SCIENCE PUBLISHERS B.V. AMSTERDAM, NL

## Description

The invention is concerned with a new scalable process for the preparation of compounds of formula I 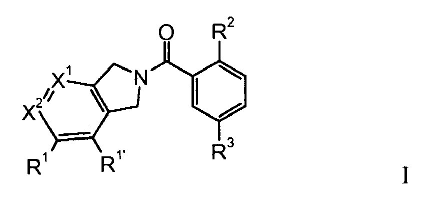 wherein
- X¹: is N or C;
- X²: is C, or when X¹ is C, X² is C or N;
- R¹ and R^{1'}: are independently of each other hydrogen, hydroxy, halogen, C₁-C₆ alkyl optionally substituted by halogen or hydroxy, C₁-C₆ alkoxy optionally substituted by halogen, cycloalkyl, -CN, -NR⁷R^{7'}, -S-C₁-C₆ alkyl, -S(O)₂-C₁-C₆ alkyl, -O-(CH₂)_{y}-C₁-C₆ alkoxy, -O(CH₂)_{y} C(O)N(C₁-C₆ alkyl)₂, -C(O)-C₁-C₆ alkyl, -C(O)O-C₁-C₆ alkyl, -C(O)-NH-C₁-C₆ alkyl, or -C(O)-N(C₁-C₆ alkyl)₂;
- y: is 1, 2, 3 or 4;
- R²: is halogen, lower alkyl optionally substituted by halogen or hydroxy, lower alkoxy optionally substituted by halogen, cycloalkyl, cyclic amine, -NR⁷R^{7'}, heterocycloalkyl, aryl or 5-or 6-membered heteroaryl, containing one, two or three heteroatoms, selected from the group consisting of oxygen, sulphur or nitrogen;
- R³: is -S(O)₂-lower alkyl, -S(O)₂NH-lower alkyl, -NO₂ or -CN; and
- R⁷, R^{7'}: are independently of each other hydrogen or lower alkyl.

Compounds of formula I are glycin transporter inhibitors and are suitable for the treatment of neurological and neuropsychiatric disorders. The majority of diseases states implicated are psychoses, schizophrenia (Armer RE and Miller DJ, 2001, Exp. Opin. Ther. Patents, 11 (4): 563-572), psychotic mood disorders such as severe major depressive disorder, mood disorders associated with psychotic disorders such as acute mania or depression associated with bipolar disorders and mood disorders associated with schizophrenia, (Pralong ET et al., 2002, Prog. Neurobiol., 67: 173-202), autistic disorders (Carlsson ML, 1998, J. Neural Transm. 105: 525-535), cognitive disorders such as dementias, including age related dementia and senile dementia of the Alzheimer type, memory disorders in a mammal, including a human, attention deficit disorders and pain (Armer RE and Miller DJ, 2001, Exp. Opin. Ther. Patents, 11 (4): 563-572).

A method for the preparation of compounds of formula I is described in WO 2006/082001. This method comprises 9 steps and is not suitable for large-scale production. It has several drawbacks such as the price and the availability of the starting materials; it involves untechnical steps such as a *Sandmeyer* reaction, a low overall yielding reduction step as well as chromatographic purifications of the intermediates.

Furthermore, EP 0275064 describes present hydrogenolysis step e) for producing intermediate compounds of formula II. But EP 0275064 describes that halogen atoms are used as leaving groups instead of mesylate groups in the cyclisation step d). Present steps a) to c) are not mentioned in this document.

Document Bioorg. & Medicinal Chemistry, Vo. 6, 1998, 143 - 149 refers to steps b) and c) of the present invention, wherein it is described a different cyclisation reaction. The formation of an isoindolinium of formula 6 is not suggested.

Database-accession No. 5256692, reaction ID describes only present step a). The invention concerns thus a new process for the preparation of compounds of formula I, especially for the preparation of compounds of formula Ia 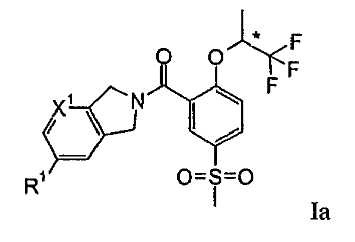 wherein
- X¹: is N or C, preferably N;
- R¹: hydrogen, hydroxy, fluoro, chloro, lower alkyl optionally substituted by halogen or hydroxy, cycloalkyl, lower alkoxy optionally substituted by halogen, -CN, -NR⁷R^{7'}, -S-lower alkyl, -S(O)₂-lower alkyl, -O-(CH₂)_{y}-lower alkoxy, -O(CH₂)_{y}C(O)N(lower alkyl)₂, -C(O)-lower alkyl, -C(O)O-lower alkyl, -C(O)-NH-lower alkyl, or -C(O)-N(lower alkyl)₂;
- y: is 1, 2, 3 or 4;
- R⁷, R^{7'}: are as defined above; and
- *: denotes a chirality center.

According to the present invention compounds of formula I, and especially compounds of formula Ia, wherein R¹ is trifluoromethyl can be prepared in high yields and a scalable manner as depicted in reaction scheme 1 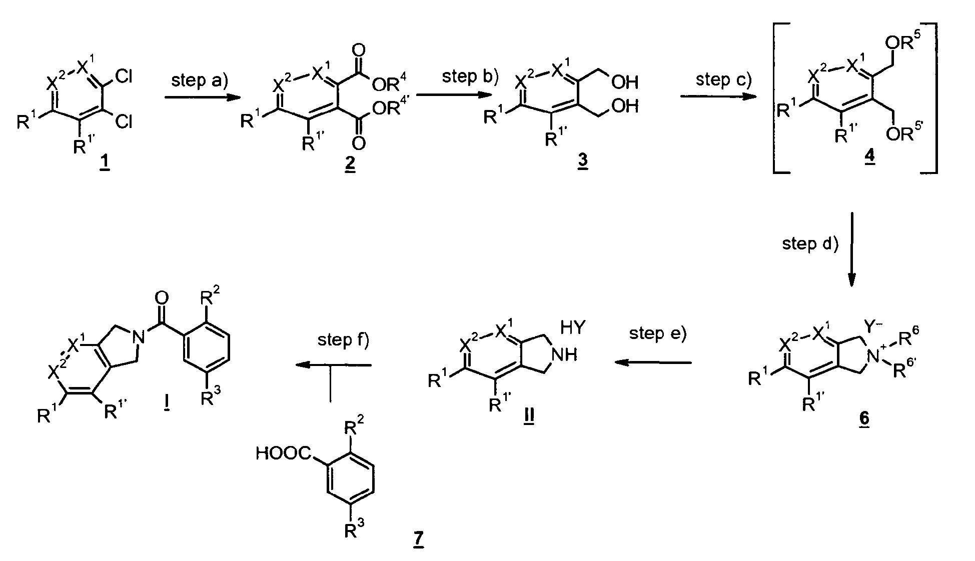 wherein the symbols X¹, X², R¹, R^{1'}, R² and R³ are as defined above.
- R⁴, R^{4'}: are independently C₁-C₆-alkyl;
- R⁵, R^{5'}: are independently a leaving group such as nosylate, tosylate, mesylate;
- R⁶ and R^{6'}: are independently of each other hydrogen, diphenylmethyl, benzyl, diallyl or allylbenzyl;
- HY: is an acid which forms a salt, such as hydrochloric acid, hydrofluoric acid, hydrobromic acid, or hydriodic acid, methane sulfonic acid, toluene sulfonic acid or 4-nitrotoluene sulfanic acid;
- Y⁻: is an anion of an acid HY.

Compounds of formula 7 are available commercially or can be prepared according to methods described in the art, for example in WO 2006/082001.

Compound of formula **7**, wherein R² is -O-CH(CH₃)-CF₃ and R³ is -SO₂CH₃ can be prepared by enzymatic reduction of 1,1,1-trifluoro-propan-2-one with Baker's Yeast to yield the optically active (S)-1,1,1-trifluoro-propan-2-ol, which is then reacted with a fluoro substituted aryl group in the presence of a base and in an appropriate solvent as described in WO 2008/107334.

In the alternative (S)-1,1,1-trifluoro-propan-2-ol can be prepared *via* a conventional enzymatic racemate resolution approach and further reacted as described in WO 2008/107334.

Yet another method to prepare compound **7** wherein R² is -O-CH(CH₃)-CF₃ and R³ is -SO₂CH₃, is by asymmetric hydrogenation of 1,1,1-trifluoro-propan-2-one and subsequent coupling with 2-fluoro-5-methane sulfonyl-benzoic acid. The asymmetric hydrogenation of 1,1,1-trifluoro-propan-2-one can be carried out with a Ru biphosphine complex such as (*S*)-3,5-*t*Pe-MeOBIPHEP (Phosphine, (6,6'-dimethoxy[1,1'-biphenyl]-2,2'-diyl)bis[bis(3,5-di-*tert*.-pentyl-phenyl)-) or (*S'*)-3,5-*t*Bu-MeOBIPHEP (Phosphine, (6,6'-dimethoxy[1,1'-biphenyl]-2,2'-diyl)bis[bis(3,5-di-*tert*.-butyl-phenyl)-) as described in EP 06/117928.9 filed on July 27, 2006.

The process according to the invention is a 5+1 step synthesis of compounds of formula I, especially of a compound of formula Ia. The key intermediate of formula II is prepared in a 4 step synthesis according to the invention and coupled to a compound of formula 7. The reaction steps of this synthesis are described in more detail below:

### step a) Carbonylation of a compound of formula 1 to the diester of formula 2

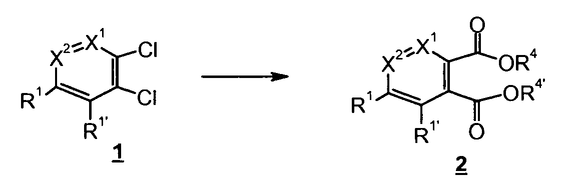

wherein R¹, R^{1'}, R⁴, R^{4'}, X¹ and X² are defined as above.
In one embodiment R⁴ and R^{4'} are independently of each other lower alkyl, R^{1'} is hydrogen and X² is carbon (C).

The double alkoxycarbonylation of a dichloro derivative of formula **1** to the dialkyl ester **2** is carried out with CO in the presence of a catalyst such as PdCl₂.dppf.CH₂Cl₂ (dppf being 1,1'-BIS(DIPHENYLPHOSPHINO)FERROCENE, PdCl₂.dppf PdCl₂.(PPh₃)₂, (PPh₃ being triphenylphosphine) Pd(OAc)₂(PPh₃)₂ (Pd(OAc)₂ being palladium diacetate), Pd(OAc)₂.dppf, preferred is PdCl₂.dppf.CH₂Cl₂ and in the presence of a base such as triethyl amine, Na₂CO₃, preferred is sodium acetate optionally water free in a lower alcohol such as methanol or ethanol. The reaction temperature is 80 to 150°C, preferred is 120°C, the pressure from 5 to 100 bar, preferred 15 bar, the reaction time is from 2.5 to 24 hrs, preferred 18 hrs.

In one embodiment the catalyst is used as a complex (0.1-0.3 mol-%) rather than being prepared *in situ,* where the amount of metal precursor is 0.5 mol-% Pd(OAc)₂ and the ligand dppf is 3 mol-%.

For compounds of formula I, wherein X¹ and X² are C, this step can be omitted and the readily available substituted phthalic acid derivatives can be used as starting materials in step b).

### step b) Reduction of the diester 2 to the diol 3.

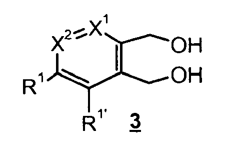

wherein R¹, R^{1'}, X¹ and X² are as defined above, preferably wherein R^{1'} is hydrogen X² is C and X¹ is N and R¹ is a s defined above..

The reduction of the diester of formula **2** can be carried out in the presence of reduction reagent such as NaBH₄ in acetic acid, DIBAH (diisobutyl aluminum hydride), LiBH₄ or with LiAlH₄, and optionally an alkali or earth alkali salts such as BaCl₂, CaCl₂, MgCl₂, MnCl₂, FeCl₂, YCl₃, CeCl₃, SrCl₂, ZnCl₂, ZrCl₄, LiCl.

In one embodiment of the invention however, reduction of a diester of formula **2** wherein R¹ is trihaloalkyl and R^{1'} is hydrogen is carried out in the presence of a reduction agent as NaBH₄ in acetic acid, DIBAH (diisobutyl aluminum hydride), LiBH₄ or LiAlH₄ and an alkali or earth alkali salts such as BaCl₂, CaCl₂, MgCl₂, MnCl₂, FeCl₂, YCl₃, CDCl₃, SrCl₂, ZnCl₂, ZrCl₄, LiCl·MgCl₂ and/or CaCl₂. The reaction is carried out in solvents such as lower alcohol, for example in methanol or ethanol or in a mixture of lower alcohol/ether for example in ethanol/tetrahydrofuran.

In one embodiment of the invention the reduction of compounds of formula **2**, wherein R¹ is trihaloalkyl and R^{1'} is hydrogen, is carried with NaBH₄ in the presence of MgCl₂ in ethanol or ethanol/tetrahydrofuran at a temperature of 0°C to 50°C, preferably at a temperature of 25°C - 30°C.

Work-up of the reaction mixture is carried out by addition of a base such as aqueous bicarbonate, elimination of the solvent, followed by extraction with an acid such HCl or citric acid. Formation of the corresponding boric acid ester is avoided for example by complete removal of the solvent, for example ethanol and by adjusting the pH to > 7.

### step c) Formation of the diol derivative 4

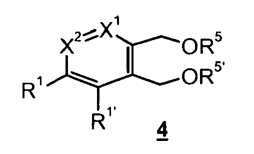

wherein R¹, R^{1'}, R⁵, R^{5'}, X¹ and X² are as defined above, preferably wherein R^{1'} is hydrogen X² is C and X¹ is N and R¹ is a s defined above.

The diol derivative **4** is formed according to methods known in the art, for example in a solvent wherein the salts formed during the reaction are insoluble and easily removable by filtration. The reaction can be carried in presence of a base such diisopropylethyl amine (DIPEA), triethylamine (NEt₃) in polar solvents such as ethylacetate at temperatures from -20°C to 40°C. For example the reaction can be carried out using mesylchloride and NEt₃ (triethylamin) in ethyl acetate at a temperature of about 0°C. The resulting salts are filtered off and the filtrate containing the compound of formula **4** can be used in the cyclization step d) without further purification. Complete conversion of the diol to its derivative **4** can be reached from water free diol.

### step d) Cyclization with an amino derivative of formula 5

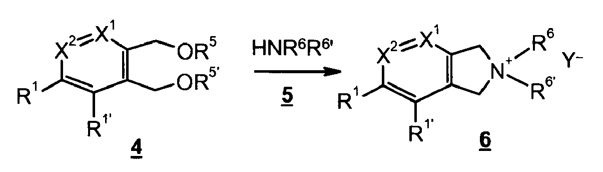

wherein
- R¹, R^{1'}, X¹ and X²: are as defined above,
- R⁶, R^{6'}: are independently of each other hydrogen, an amino protecting groups such as benzyl, diphenylmethyl or the like; and
- Y⁻: is an anion such as Cl⁻, F⁻, Br⁻, I⁻, mesylate, tosylate or nosylate,
preferably wherein R^{1'} is hydrogen X² is C and X¹ is N and R¹ is a s defined above.

For compounds of formula **6**, wherein R^{6'} is hydrogen, the free acid or the corresponding salt can be isolated.
for R^{6'} = H 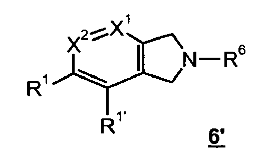 or a salt thereof

In one embodiment the amino derivative **5** is diphenylmethyl amine (R⁶ is diphenylmethyl and R^{6'} is hydrogen). The cyclisation to form the diphenylmethyl derivative **6** the reaction is carried out at a temperature of 60°C to 100°C in a solvent such as tetrahydrofuran, methanol or dimethylformamide or mixtures thereof, in the presence of a base such as Hünig's base (diisopropylethylamine, DIPEA), K₂CO₃ or NEt₃.

In an other embodiment the amino derivative **5** is dibenzyl amine (R⁶ and R^{6'} are benzyl). For the preparation of the dibenzylamino derivative **6** the reaction can be carried out with dibenzylamine and in the presence of a base such as Hünig's base in tetrahydrofuran, methanol or dimethylformamide preferably in ethyl acetate preferably under reflux conditions. The pure product precipitated directly during the reaction as the corresponding salt and is stable against oxidation on air.

The purification of the product (**6**) can be done by crystallisation and/or formation of its salt.

In one embodiment the hydrochloride salt of compound (**6**) is formed using acetyl chloride in methanol.

In another embodiment the bicyclic product (**6**) can be isolated directly from the reaction mixture as its mesylate, tosylate or nosylate salt.

### step e) Hydrogenolysis of the protected amine (6) or its salt to the amine of formula II or a salt thereof:

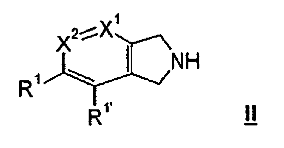

wherein R¹, R^{1'}, X¹ and X² are as defined above, preferably wherein R^{1'} is hydrogen X² is C and X¹ is N and R¹ is a s defined above.
is accomplished with hydrogen using catalytic amounts of Pd/C in a suitable solvent. Suitable solvents are for example alcohols, such as methanol, ethanol or ethers such as tatrahydrofuran, or hydrocarbons such as toluene, or mixtures thereof. Preferably the reaction is carried out at room temperature.

In an embodiment an acid such as for example hydrochloric acid is used during the hydrogenolysis and the corresponding salt of compound II is obtained.

In a further embodiment a salt, preferably the mesylate or hydrochloride salt of the compound of formula II is prepared by hydrogenation of the corresponding salt of formula 6 in the presence of a catalyst such as Pd/C (10%) in an alcohol under a hydrogen atmosphere.

The reaction is carried out at a temperature of 0°C to 50 °C.
In a further embodiment the hydrogenation of a compound of formula **6** wherein R¹ is trihaloalkyl and R^{1'} is hydrogen is carried out under 1 atmosphere of hydrogen, in a suitable solvent such as an alcohol, for example in methanol. About 1% to 10%, preferably about 2.5 % catalyst such as Pd/C or Pd(OH)₂/C is used.

step f) Reacting the compound of formula II or a salt thereof with a suitable acid of formula **7** in the presence of an activating agent such as POCl₃, (COCl)₂, SOCl₂ or any peptid coupling reagents like HATU (O-(7-Azobenzotriazol-1-yl)-1,1,3,3-tetramethyluronium Hexafluorophosphat), TBTU (2-(1H-Benzotriazol-1-yl)-1,1,3,3-tetramethyluronium Hexafluorophosphat, CDI (1,1'-Carbonyl-diimidazol, yields the desired compound of formula I.

In an embodiment a salt, preferably the hydrochloride or the mesylate salt of the compound of formula II, wherein R^{1'} is hydrogen and X² is carbon (C), is reacted with a compound of formula 7, wherein R² is alkoxy substituted by halogen.

As used herein the term "halogen" denotes fluorine, chlorine, iodine and bromine, preferably fluorine and iodine.

The term "lower alkyl" denotes a saturated straight- or branched-chain group containing from 1 to 6 carbon atoms, for example, methyl, ethyl, propyl, isopropyl, n-butyl, i-butyl, 2-butyl, t-butyl, pentyl or hexyl. Preferred lower alkyl groups are groups with 1 - 4 carbon atoms.

The term "lower alkyl optionally substituted by halogen or hydroxy" denoted lower alkyl groups as defined above which group are unsubstituted or substituted by one or several substituents individually selected from halogen as defined above or hydroxy. Examples of substituted lower alkyl groups are trifluoromethyl (CF₃), difluoromethyl (CHF₂), fluoromethyl (CH₂F), 2,2,2-trifluoroethyl (-CH₂CF₃), 2,2-difluoroethyl (-CH₂CHF₂), 2-fluoroethyl (-CH₂CH₂F), 3,3,3-trifluoropropyl (-CH₂CH₂CF₃), 2,2-difluoropropyl (-CH₂-CF₂-CH₃), 4,4,4-trifluorobutyl (-CH₂CH₂CH₂CF₃), 1-trifluoromethyl-propyl CH(CF₃)CH₂CH₃, 2,2,2-trifluoro-1,1-dimethyl-ethyl C[(CH₃)₂]-CF-₃, 2-chloroethyl (CH₂CH₂Cl, 2,2,3,3,3-pentafluoro-propyl (CH₂CF₂CF₃), 2,2,3,3-tetrafluoro-propyl (CH₂CF₂CHF₂), 2,2,2-trifluoro-1,1-dimethylethyl (C(CH₃)₂CF₃, CH(CH₃)CF₃) or 2-fluoro-l-fluoromethyl-ethyl (CH(CH₂F)CH₂F). Preferred are CH₂CF₃, CF₃ or CH(CH₃)CF₃.

The term "lower alkoxy" denotes a saturated straight- or branched-chain alkyl group containing from 1 to 6 carbon atoms as described above and which groups are connected *via* an oxygen atom.

The term "lower alkoxy, optionally substituted by halogen" denotes a lower alkyl group as defined above attached via an oxygen group, said lower alkyl group being unsubstituted or substituted by one or several substituents individually selected from halogen as defined above. Examples of such groups are trifluoromethoxy (-OCF₃), difluoromethoxy (-O-CHF₂), fluoromethoxy (-O-CH₂F), 2,2,2-trifluoroethoxy (-O-CH₂CF₃), 2,2-difluoroethoxy (-O-CH₂CHF₂), 2-fluoroethoxy (-O-CH₂CH₂F), 3,3,3-trifluoropropyloxy (-O-CH₂CH₂CF₃), 4,4,4-trifluorobutoxy (-O-CH₂CH₂CH₂CF₃), 1-trifluoromethyl-propyloxy (-O-CH(CF₃)CH₂CH₃), 2,2,2-trifluoro-1,1-dimethyl-ethoxy (-O-C[(CH₃)₂]-CF₃), 2-chloroethoxy (-O-CH₂CH₂Cl), 2,2,3,3,3-pentafluoro-propyloxy (-O-CH₂CF₂CF₃), 2,2,3,3-tetrafluoropropyloxy (-O-CH₂CF₂CHF₂), 2,2,2-trifluoro-1,1-dimethyl-ethoxy (-O-C(CH₃)₂CF₃), 2,2,2-trifluoro-1-methyl-ethoxy (-O-CH(CH₃)CF₃) or 2-fluoro-l-fluoromethyl-ethoxy (-O-CH(CH₂F)CH₂F). Preferred are -O-CH₂CF₃, -O-CF₃ or -O-CH(CH₃)CF₃.

The term "cycloalkyl" denotes a cyclic alkyl group having 3 to 8 carbon atoms such as cyclopropyl, cyclobutyl, cyclopentyl, cyclohexyl, cycloheptyl or cyclooctyl.

The term "cyclic amine" denotes a saturated carbon ring, containing from 3 to 6 carbon atoms as defined above, and wherein at least one of the carbon atoms is replaced by a heteroatom, selected from the group consisting ofN, O or S and wherein the N-atom is linked to the phenyl ring, for example piperidine, piperazine, morpholine, thiomorpholine, di-oxo-thiomorpholine, pyrrolidine, pyrazoline, imidazolidine or azetidine. Such groups can be substituted by one or more substituents, selected from the group consisting of halogen, hydroxy, phenyl, lower alkyl, lower alkoxy or =O.

As used herein, the term "heterocycloalkyl" denotes a saturated carbon ring, containing from 3 to 6 carbon atoms, and wherein at least one of the carbon atoms is replaced by a heteroatom, selected from the group consisting of N, O or S. Examples of such groups are tetrahydropyran-2, 3 or 4-yl, tetrahydrofuran-2 or 3-yl or oxetan-3-yl, [1,4]dioxin-2-yl.

The term "aryl" denotes a one or two membered aromatic carbon ring, for example phenyl, benzyl or naphthyl.

The term "5 or 6-membered heteroaryl" denotes for example furanyl, thienyl, pyrrolyl, imidazolyl, pyrazolyl, triazolyl, thiazolyl, isothiazolyl, isoxazolyl, pyridinyl, pyrazinyl or pyrimidinyl.

The term "pharmaceutically acceptable acid addition salts" embraces salts with inorganic and organic acids, such as hydrochloric acid, hydrobromic acid, hydrofluoric acid, hydriodic acid, nitric acid, sulfuric acid, phosphoric acid, citric acid, formic acid, fumaric acid, maleic acid, acetic acid, succinic acid, tartaric acid, methanesulfonic acid or p-toluenesulfonic acid.

The term "leaving group" denotes groups such as Cl⁻, Br⁻, I⁻, nosyl⁻ (nosylate, 3-nitrobenzenesulfonate, 3-NO₂-C₆H₄-SO₃⁻), mesyl⁻ (mesylate, methylsulfonyl, CF₃SO₃⁻) or tosyl⁻ (tosylate, p-toluenesulfonyl, 4-(CH₃)-C₆H₄-SO₃⁻).

According to the invention the process for the preparation of compounds of formula I 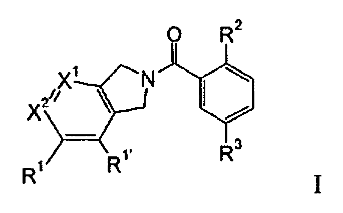 wherein
- X¹: is N or C;
- X²: is C, or when X¹ is C, X² is C or N;
- R¹ and R^{1'}: are independently of each other hydrogen, hydroxy, halogen, lower alkyl optionally substituted by halogen or hydroxy, lower alkoxy optionally substituted by halogen, cycloalkyl, -CN, -NH₂, -S-lower alkyl, -S(O)₂-lower alkyl, -O-(CH₂)_{y}-lower alkoxy, -O(CH₂)_{y}C(O)N(lower alkyl)₂, -C(O)-lower alkyl, -C(O)O-lower alkyl, -C(O)-NH-lower alkyl, or -C(O)-N(lower alkyl)₂.
- y: is 1, 2, 3 or 4;
- R²: is halogen, lower alkyl optionally substituted by halogen or hydroxy, lower alkoxy optionally substituted by halogen, cycloalkyl, -NR⁷R^{7'}, cyclic amine, heterocycloalkyl, aryl or 5-or 6-membered heteroaryl, containing one, two or three heteroatoms, selected from the group consisting of oxygen, sulphur or nitrogen.
- R³: is -S(O)₂-lower alkyl, -S(O)₂NH-lower alkyl, -NO₂ or -CN
comprises

### a) coupling a compound of formula II

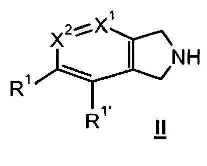

or a salt thereof
with a compound of formula 7 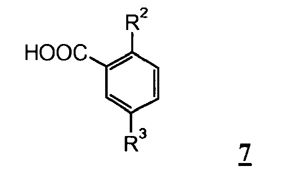 wherein R¹, R^{1'}, X¹, X², R² and R³ are as defined above,
the reaction is carried out in the presence of an activating agent such as POCl₃, (COCl)₂ or the like.

In an embodiment the process according to the invention is used for the preparation of a compound of formula Ia 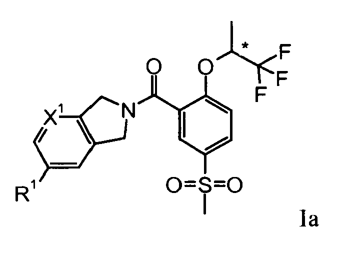 wherein
- X¹: is N or C, preferably N;
- R¹: hydrogen, hydroxy, fluoro, chloro, lower alkyl optionally substituted by halogen or hydroxy, cycloalkyl, lower alkoxy optionally substituted by halogen, -CN,-NR⁷R^{7'}, -S-lower alkyl, -S(O)₂-lower alkyl, -O-(CH₂)_{y}-lower alkoxy,-O(CH₂)_{y}C(O)N(lower alkyl)₂, -C(O)-lower alkyl, -C(O)O-lower alkyl, -C(O)-NH-lower alkyl, or -C(O)-N(lower alkyl)₂;
- y: is 1, 2, 3 or 4; and
- *: is a chirality center.

In an embodiment the process according to the invention is used in the preparation of compounds of formula Ia wherein R¹ is alkyl substituted by halogen, especially trifluoromethyl; R² is -O-CH(CH₃)CF₃ and R³ is -S(O)₂-lower alkyl.

In another embodiment the process for the preparation of a compound of formula II or a salt thereof comprises
a) carbonylation of a compound of formula **1** to the diester of formula **2** 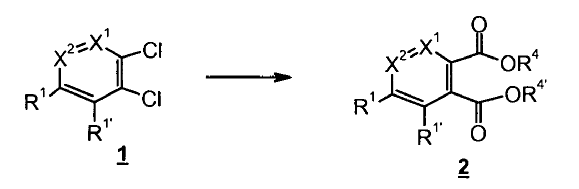 wherein R¹, R^{1'}, R⁴ and R^{4'} and X¹ and X² are defined as above, preferably R⁴ and R^{4'} are independently of each other lower alkyl, R^{1'} is hydrogen and X² is CH, in the presence of CO and a catalysator;
b) reduction of the diester **2** to the diol **3**. 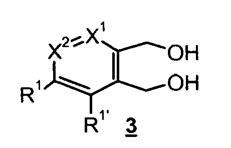 wherein R¹, R^{1'}, X¹ and X² are as defined above, preferably wherein R^{1'} is hydrogen X² is C and X¹ is N and R¹ is a s defined above.
   in the presence of reduction reagent such as NaBH₄ in acetic acid, DIBAH (diisobutyl aluminum hydride), Redal (bis(2-methoxyethoxy)aluminium hydride) LiBH₄ or LiAlH₄ and optionally an alkali and/or alkaline earth or transition metal salt. The alkali or alkaline earth salt is preferably BaCl₂, CaCl₂, MgCl₂, MnCl₂, FeCl₂, YCl₃, CeCl₃, SrCl₂, ZnCl₂, ZrCl₄, LiCl·MgCl₂ and/or CaCl₂, especially CaCl₂.
c) formation of the diol derivative **4** 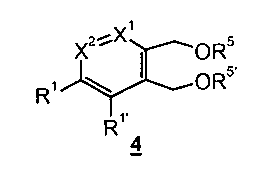 wherein R⁵ and R^{5'} are as defined above, preferably wherein R^{1'} is hydrogen X² is C and X¹ is N and R¹ is a s defined above.
   the diol **3** is reacted in presence of a base, in a solvent wherein the salts formed during the reaction are insoluble and easily removable by filtration.
d) Cyclization with an amino derivative of formula **5** 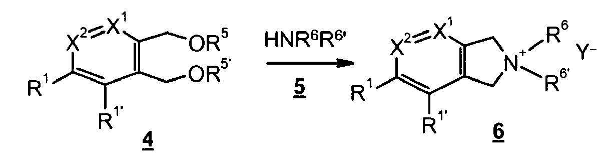
   - R¹, R^{1'}, X¹ and X²: are as defined above,
   - R⁶, R^{6'}: are independently of each other hydrogen, an amino protecting group such as benzyl, diphenylmethyl or the like; and
   - Y⁻: is an anion such Cl⁻ or meylate, tosylate or nosylate,
   preferably wherein R^{1'} is hydrogen X² is C, X¹ is N and R¹ is a s defined above.
   with an amino derivative of formula **5** at a temperature 60°C to 100°C, in the presence of a base such as Hünig's base (diisopropylethylamine, DIPEA), K₂CO₃ or triethyl amine.
   In one embodiment the hydrochloride salt of compound (**6**) is formed using acetyl chloride in methanol.
   In another embodiment the bicyclic product (**6**) can be isolated directly from the reaction mixture as its mesylate, tosylate or nosylate salt.
   For compounds of formula (**6**), wherein R^{6'} is hydrogen, the free acid or the corresponding salt can be isolated.
e) Hydrogenolysis of the protected amine (**6**) or its salt to the amine formula II or a salt thereof: 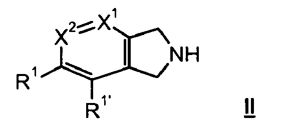 wherein R¹, R^{1'}, X¹ and X² are as defined above,
   under hydrogen in the presence of catalytic amounts of Pd/C, optionally in the presence of an acid to yield the corresponding salt.

In one embodiment a salt of a compound of formula II is prepared by hydrogenation of a compound of formula **6**, wherein R¹ is lower alkyl substituted by halogen, R^{1,} and R^{6,} are hydrogen and X² is carbon in the presence a catalytic amount of Pd/C and hydrochloric acid.

### Examples

### 1. Synthesis of 2,3-pyridine dicarboxylic acid-5-trifluoromethyl diethyl ester

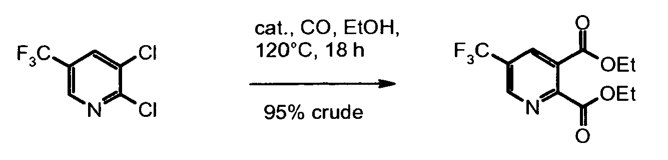

A 200 1 autoclave was charged with a solution of 14.6 kg 2,3-dichloro-5-trifluoromethyl pyridine (67.6 mol) in 60.0 1 ethanol and was treated at room temperature under argon with a suspension of 73 g palladium(II)dichloride 1,1'-bis(diphenylphosphino)-ferrocene (89.4 mmol) and 13.6 kg sodium acetate (165.8 mol) in 54 1 ethanol. The autoclave was sealed, the carbon monoxide pressure set at 15 bar and the carbonylation was run under stirring at 120°C for 18 h, then cooled to 22°C. The autoclave was cooled to room temperature then vented.

The black suspension was filtered and the vessel rinsed twice with 50 1, in total with 100 1 ethanol. The filtrate was evaporated under reduced pressure at 50°C and the residue dissolved in a mixture of 30 1 ethyl acetate and 15 l toluene. The solution was washed with 30 1 water and after phase separation the organic phase was evaporated under reduced pressure at 50°C to yield 18.7 kg (95%) of 2,3-pyridine dicarboxylic acid-5-trifluoromethyl diethyl ester as an oil.

MS (EI): 292 (M+H⁺,17), 272 (M-F, 12), 246 (32), 218 (100), 191 (17), 174 (47), 147 (81).

### 2.1. Synthesis of (2-hydroxymethyl-5-trifluoromethyl-pyridin-3-yl)-methanol

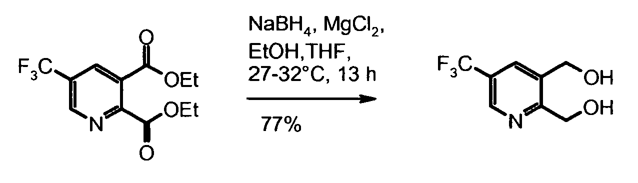

A suspension of 1.63 kg MgCl₂ (16.8 mol) in 14.5 1 tetrahydrofuran was treated at Tₘₐₓ = 10°C over 35 min with 11.8 1 ethanol (exothermic reaction). The reaction mixture was vigorously stirred and treated in portions (over 30-60 min, temperature controlled) with a suspension of 1.3 kg sodium borohydride (32.99 mol) in 6.7 1 tetrahydrofuran. A very exothermic reaction ensued, accompanied by foaming. The mixture was warmed to 28°C and stirred at this temperature over 20 min. To the suspension was added over 35 min at T = 27 - 32°C a solution of 1.69 kg 2,3-pyridine dicarboxylic acid-5-trifluoromethyl diethyl ester (5.7 mol) in 5.1 l ethanol. The yellow-orange suspension was stirred for 13 h at T = 27 - 32°C.

The yellow reaction mixture was added in portions over 18 min to a solution of 5.9 kg sodium bicarbonate (81.9 mol) and 68 l water resulting in an exothermic reaction and gas evolution. The yellow suspension was stirred for 18 min, then all of the organic solvent was removed by evaporation under reduced pressure (400 - 50 mbar) at 60°C. The total volume was adjusted with water to 70 l. The suspension was treated under vigorous stirring with 10.5 kg citric acid (54.3 mol) dissolved in 8.4 l water to adjust the pH to 7 (foaming). To the yellow solution was added 40 l *tert*.-butyl-methyl ether. After extraction and phase separation, the water phase was extracted twice with 30 l, in total with 60 l *tert*.-butyl-methyl ether. The combined organic phases were dried over 15 kg Na₂SO₄ and the solvent was evaporated at 60°C and 750 - 10 mbar yielding 913.0 g (76.9%) of (2-hydroxymethyl-5-trifluoromethyl-pyridin-3-yl)-methanol as a yellow-red oil
MS (EI): 208 (M+H⁺, 13), 289 (54), 161 (100).

### 2.2 Preparation of (2-Fluoro-6-hydroxymethyl-phenyl)-methanol starting from 3-Fluoro-phthalic acid dimethyl ester

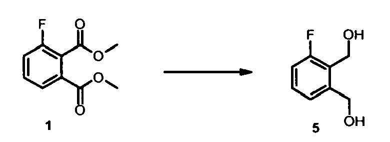

In a 10 ml two necked round bottom flask equipped with a thermometer, magnetic stirrer and an inert gas supply, 1.20 ml of a 3.5M solution of sodium dihydrido-bis(2-methoxy-ethoxy)aluminate (4.2 mmol) in toluene was cooled to 0-5°C. A solution of 212.0 mg (1.0 mmol) 3-fluorophthalic acid dimethylester in 2.2 ml tetrahydrofuran was added dropwise over 5 min at 0-5°C. The reaction mixture was stirred for 1.5 h at 0-5°C. Over 5 min a solution of 1.05 ml brine and 1.05 ml 2M sodium hydroxide was added dropwise at 0-5°C. The reaction mixture was extracted with 3.0 ml tert-butyl methyl ether, the organic phase was separated and dried with sodium sulfate, filtered and evaporated at 40°C and 190-9 mbar yielding 110.0 mg (70%) of (2-fluoro-6-hydroxymethyl-phenyl)-methanol as an off white solid.
MS (EI): 138 (100), 137 (78), 109 (57)

### 2.3. Preparation of (2-fluoro-6-hydroxymethyl-phenyl)-methanol (5) starting from fluorophthalic acid

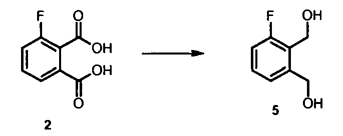

In a 500 ml four necked round bottom flask equipped with a thermometer, mechanic stirrer and an inert gas supply 18.41 g 3-fluorophthalic acid (100 mmol) was dissolved in 92 ml tetrahydrofuran and cooled to 0-5°C. Over 93 min, 143 ml of a 3.5M solution of sodium dihydrido-bis(2-methoxyethoxy)aluminate in toluene (500 mmol) were added. The reaction mixture was stirred for 1 h at 0-5°C and then 2 h at room temperature. The light orange solution was cooled to 0-5°C, the over 13 min a solution of 125 ml brine and 125 ml 2M sodium hydroxide was added dropwise. The organic phase was separated and the aqueous solution was extracted with 240 ml tert-butyl methyl ether, the combined organic phases were dried over 280 g sodium sulfate, filtered and evaporated in a rotary evaporator at 40°C and 300-10 mbar. The residue was treated three times with a solution of 10 ml tert-butyl methyl ether and 50 ml toluene and evaporated at 40°C and 300-10 mbar to obtain 14.25 g (91%) of (2-fluoro-6-hydroxymethylphenyl)-methanol as beige crystals.
MS (EI): 138 (100), 137 (79), 109 (57)

### 2.4. Preparation of (2-fluoro-6-hydroxymethyl-phenyl)-methanol (5) starting from fluoro phthalic acid anhydride

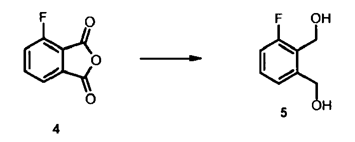

In a 10 ml two necked round bottom flask equipped with a thermometer, magnetic stirrer and an inert gas supply, 1.20 ml of a 3.5M solution of sodium dihydrido-bis(2-methoxyethoxy)aluminate in toluene (4.2 mmol) was cooled to 0-5°C. A solution of 166.1 mg 4-fluoro-isobenzofuran-1,3-dione (1.0 mmol) in 1.60 ml tetrahydrofuran was added dropwise over 5 min at 0-5°C. The reaction mixture was stirred for 4.5 h at 0-5°C. In the course of 5 min a solution of 1.05 ml brine and 1.05 ml 2M sodium hydroxide was added dropwise at 0-5°C. The reaction mixture was extracted with 3.0 ml tert-butyl methyl ether, the organic phase was separated and dried over sodium sulfate, filtered and evaporated at 40°C 190-9 mbar yielding 104.0 mg (68%) of (2-fluoro-6-hydroxymethyl-phenyl)-methanol as an off white solid.
MS (EI): 138 (100), 137 (96), 109 (64)

### 2.5. Preparation of (2-fluoro-6-hydroxymethyl-phenyl)-methanol starting from fluoro phthalic acid mono ethyl ester

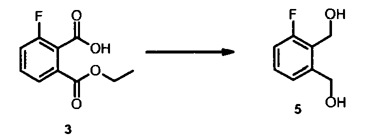

In a 25 ml two necked round bottom flask equipped with a thermometer, magnetic stirrer and an inert gas supply, 424.4 mg 3-fluorophthalic acid 1-ethyl ester (2.0 mmol) was dissolved in 2.12 ml tetrahydrofuran and cooled to 0-5°C. Over 10 min, 2.86 ml of a 3.5M solution of sodium dihydrido-bis(2-methoxyethoxy)aluminate in toluene (10.0 mmol) were added dropwise. The reaction mixture was stirred for 1h at 0-5°C and then 2 h at room temperature. The light orange solution was cooled to 0-5°C, a solution of 2.5 ml brine and 2.5 ml 2M sodium hydroxide was added dropwise over 13 min. The reaction mixture was extracted with 4.8 ml tert-butyl methyl ether. The organic phase was separated and the aqueous solution was extracted with 3.2 ml tert-butyl methyl ether, the combined organic phases were dried over 1.8 g sodium sulfate, filtered and evaporated in a rotary evaporator at 40°C and 300-10 mbar. The residue was treated three times with a solution of 0.2 ml tert-butyl methyl ether and 1.2 ml toluene and evaporated at 40°C and 300-10 mbar providing 256 mg (82%) of (2-fluoro-6-hydroxymethyl-phenyl)-methanol (5) as an off white crystals.
MS (EI): 138 (100), 137 (94), 109 (57)

### 3.1. a) Syntheses of 6-benzhydryl-3-trifluoromethyl-6,7-dihydro-5H-pyrrolo[3,4-b] pyridine

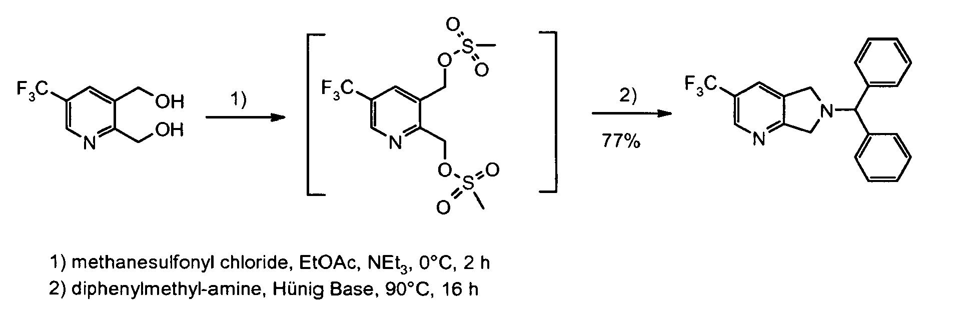

A 1.5 1 four-necked round bottom flask equipped with a thermometer, a dropping funnel, an intensive cooler, a mechanical stirrer and an inert gas supply was charged with a solution of 69 g diol (2-hydroxymethyl-5-trifluoromethyl-pyridin-3-yl)-methanol (333.1 mmol) and 900 ml ethyl acetate which was cooled to 0°C and treated over ca. 10 min at T = 0 - 5°C with 83.7 g methanesulfonyl chloride (730.7 mmol). The brown solution was stirred at T = 0 - 5°C for 10 min and treated over 60 min at T = 0 - 5°C with a solution of 85.1 g triethylamine (836.4 mmol) in 200 ml ethyl acetate (exothermic reaction). The yellow suspension was stirred for 2 h at T = 0 - 5°C then filtered and the residue was washed with 200 ml ethyl acetate. The combined dimesylate filtrate was treated at T = 5 - 10°C with 61.0 g diphenylmethyl amine (333.0 mmol) and 87.8 g *Hünig's* base (666.0 mmol). The reaction mixture was heated to T = 76 - 80°C and stirred at this temperature for 2.5 h and over night under reflux.

The mixture was poured in one portion into 940 ml water (exothermic reaction), vigorously stirred for 10 min and then the phases separated. The organic phase was washed with 900 ml half saturated NaCl solution and dried over Na₂SO₄. The solvent of the organic phase was evaporated (60°C, 300 to 10 mbar) yielding 115 g of crude 6-benzhydryl-3-trifluoromethyl-6,7-dihydro-5H-pyrrolo[3,4-b] pyridine as a beige residue containing traces of ethyl acetate.

The crude product was treated with 500 ml isopropanol and stirred at 60°C for 10 min. The solvent of the light brown suspension was evaporated again and the procedure repeated a second time. The residue was dissolved at 80°C in 1.65 1 isopropanol and filtered over charcoal. The charcoal was rinsed with 170 ml isopropanol and the total volume concentrated to 900 ml. The solution was treated at T = 75 - 80°C with 300 ml water. The suspension was cooled down over 2 h to room temperature and stirred over night at this temperature. The thick light brown suspension was then stirred for 1 h at 40°C, cooled over 5 h to 0°C and stirred at this temperature over night. The suspension was filtered, the crystals rinsed with 340 ml isopropanol and dried at 50°C and 10 mbar over two days to yield 73.5 g (62.3%) of 6-benzhydryl-3-trifluoromethyl-6,7-dihydro-5H-pyrrolo[3,4-b] pyridine as white crystals
MS (turbo spray): 355 (M+H⁺, 100), 167 (37).

### 3.1.b) Synthesis of 6-benzhydryl-3-trifluoromethyl-6,7-dihydro-5H-pyrrolo[3,4-b]pyridine hydrochloride salt

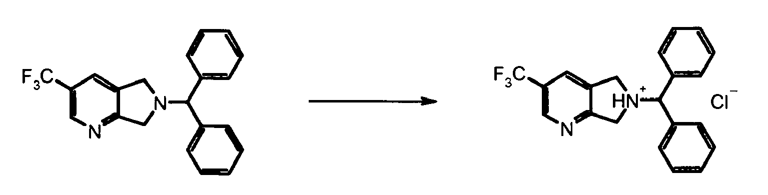

A 350 ml four-necked round bottom flask equipped with a thermometer, a dropping funnel, an intensive cooler, a mechanical stirrer and an inert gas supply was charged with 117 ml methanol which was treated over 5 min at T = 15 - 30°C with 2.6 ml acetyl chloride (36.3 mmol) (exothermic reaction). The colorless solution was stirred for 10 min at room temperature and treated in one portion with 11.7 g (33.0 mmol) of 6-benzhydryl-3-trifluoromethyl-6,7-dihydro-5H-pyrrolo[3,4-b] pyridine. The powder funnel was rinsed with 20 ml methanol and the clear yellow solution was stirred at room temperature for 15 min. The solvent was evaporated at 60°C under reduced pressure to a total volume of 20 ml and 210 ml *tert*.-butyl-methyl ether was added at 60°C. The mixture was heated to reflux and stirred at this temperature for 40 min then cooled over 1 h to room temperature. The yellow suspension was filtered, the crystals rinsed with 22 ml *tert*.-butyl-methyl ether and dried at room temperature and <10 mbar for 12 h yielding 11.5 g (89.2%) of 6-benzhydryl-3-trifluoromethyl-6,7-dihydro-5H-pyrrolo[3,4-b] pyridine hydrochloride salt as light-yellow crystals.
MS (EI): 355 (M+H⁺, 100), 167 (9).

### 3.2. Synthesis of 6,6-dibenzyl-3-trifluoromethyl-6,7-dihydro-5H-pyrrolo[3,4-b]pyridin-6-ium methanesulfonate

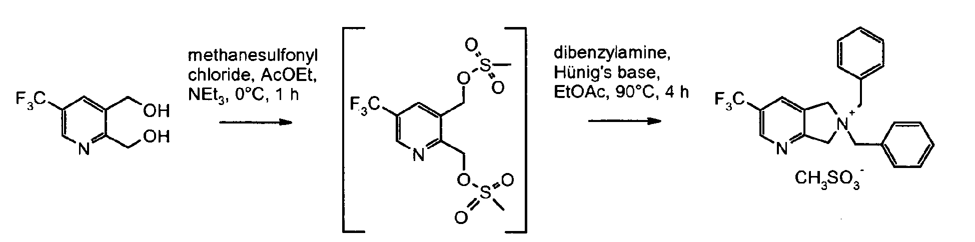

A 500 ml four-necked round bottom flask equipped with a thermometer dipping into the reaction mixture, a dropping funnel, an intensive condenser, a mechanical stirrer and an inert gas supply was charged with a solution of 10.36 g diol (2-hydroxymethyl-5-trifluoromethyl-pyridin-3-yl)-methanol, 50.0 mmol) and 180 ml ethyl acetate. The solution was cooled to 0 - 5°C and treated with 8.5 ml methanesulfonyl chloride (110.0 mmol), T rose from 1.0 to 2.0°C. The brown solution was stirred at T = 0 - 5°C and treated within 70 min at T = 0 - 5°C with a solution of 17.4 ml triethylamine (125.0 mmol) in 19 ml ethyl acetate (exothermic reaction). The yellow suspension was stirred for 0.5 h at T = 0 - 5°C and filtered. The residue was washed with 65 ml ethyl acetate. The combined dimesylate filtrates were treated at T = 5 - 10°C with 9.6 ml dibenzylamine (50.0 mmol), 9.4 ml *Hünig's* base (55.0 mmol) and 13.2 ml ethanol. The reaction mixture was heated to reflux (T = 74°C) and stirred at this temperature for 2 h. To the light brown suspension, a further 0.5 ml dibenzylamine (2.5 mmol) was added and refluxed for another 2.5 h to complete the reaction.

The light brown suspension was cooled to room temperature over 2 h, stirred for 16 h at room temperature, then cooled to 0 - 5°C and stirred for 2 h at this temperature. The light brown suspension was filtered over a pre-cooled (0 - 5°C) glass filter funnel G3. The filter cake was washed with a pre-cooled (0 - 5°C) mixture of 80 ml ethyl acetate and 5 ml ethanol. The white crystals were dried in a rotary evaporator at 40°C/3 mbar for 6 h yielding 16.2 g (69.6%) of 6,6-dibenzyl-3-trifluoromethyl-6,7-dihydro-5H-pyrrolo[3,4-b]pyridin-6-ium methanesulfonate as white crystals
MS (EI): 369 (M⁺, 100), 277 (8).

### 3.3. a) Preparation of Methanesulfonic acid 2-fluoro-6-methanesulfonyloxymethyl-benzyl ester

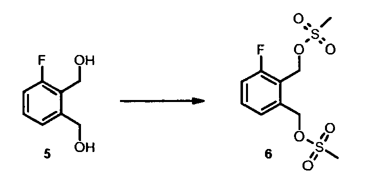

In a 200 ml two necked round bottom flask equipped with a thermometer, a magnetic stirrer and an inert gas supply 10.15 g (2-fluoro-6-hydroxymethyl-phenyl)-methanol (65.0 mmol) was dissolved in 208 ml ethyl acetate, cooled to 0-5°C, over 5 min, 11.1 ml methanesulfonyl chloride (143.0 mmol) was added dropwise. A solution of 22.6 ml (162.5 mmol) triethylamine in 25 ml ethyl acetate was then added dropwise over 38 min. The white suspension was stirred for 2 h at 0-5°C, filtered over a pre- cooled (0-5°C) glass filter funnel and washed with 65 ml pre-cooled (0-5°C) ethyl acetate yielding 325 ml solution with product.

### 3.3.b) Preparation of 2-Benzhydryl-4-fluoro-2,3-dihydro-1H-isoindole

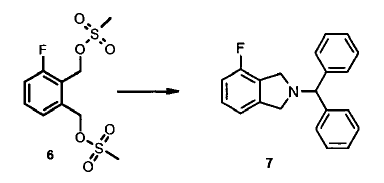

In a 500 ml two necked round bottom flask equipped with a thermometer a reflux condenser a magnetic stirrer and an inert gas supply, 325 ml methanesulfonic acid 2-fluoro-6-methanesulfonyloxymethyl-benzyl ester solution (65 mmol) in ethyl acetate, 12.3 ml aminodiphenylmethane (71.5 mmol) and 27.8 ml N-ethyldiisopropylamine (162.5 mmol) were added. The reaction mixture was heated under reflux for 6 h, cooled to room temperature and the solvent was evaporated at 40°C and 160 to 10 mbar yielding 49 g crude 2-benzhydryl-4-fluoro-2,3-dihydro-1H-isoindol as a light pink solid. The crude product was treated with 197 ml methanol and heated to reflux for 15 min. The light brown suspension was cooled over 30 min to room temperature stirred for 2 h at room temperature and cooled to 0-5°C and stirred for 30 min. The suspension was filtered over a pre-cooled glass filter funnel and the residue was washed with 66 ml cold methanol. The white crystals were dried at 40°C and 10 mbar yielding 32.4 g (83%) of 2-benzhydryl-4-fluoro-2,3-dihydro-1*H*-isoindole (7)
MS (turbo spray) 304 (M+H⁺, 39), 346 (11), 167 (100)

### 3.3. c) Preparation of 4-fluoro-2,3-dihydro-1H-isoindol hydrochloride (8)

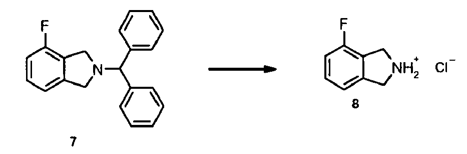

In a 250 ml four necked round bottom flask equipped with a thermometer a magnetic stirrer and an hydrogen gas supply 35 ml methanol was cooled to 0-5°C and 1.80 ml (25.3 mmol) acetylchloride was slowly added. The temperature rose to 20°C and this mixture was stirred for 15 min at room temperature, before adding 6.98 g (23.0 mmol) 2-benzhydryl-4-fluoro-2,3-dihydro-1H isoindole and 35 ml methanol. The white suspension was stirred for 10 min at room temperature and 0.70 g palladium-charcoal 10% was added. The vessel was evacuated three times and ventilated, first with argon, then with hydrogen. The black mixture was hydrogenated for 7 h at room temperature, filtered and the filter cake was washed with 20 ml methanol. The light yellow filtrate was treated again with 0.70 g palladium-charcoal 10% and hydrogenated for another 15 h at room temperature. The black suspension was filtered and the filter cake was washed with 25 ml methanol. The clear colorless filtrate was evaporated at 40°C and 300 to 10 mbar to obtain 7.34 g as white residue. The crude product was suspended in 40 ml tert-butyl methyl ether, heated to refluxed for 10 min, cooled to room temperature and stirred for 3 h at room temperature. The suspension was filtered and the filter cake was washed with 13 ml tert-butyl methyl ether. The white crystals were dried at 40°C and 10 mbar yielding 3.74 g (94%) of 4-fluoro-2,3-dihydro-1*H*-isoindole hydrochloride as white crystals.
MS (turbo spray) 138 (M+H⁺, 100), 139 (10)

### 3.4. a) same reaction as in 3.3. a)

### 3.4.b) Preparation of 2,2-Dibenzyl-4-fluoro-2,3-dihydro-1H-isoindolium methanesulfonate (9)

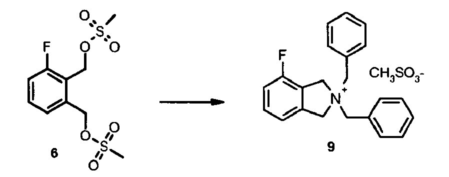

In a 50 ml two necked round bottom flask equipped with a thermometer a magnetic stirrer a reflux condenser and an inert gas supply 1.56 g methanesulfonic acid 2-fluoro-6-methanesulfonyloxymethyl-benzyl ester (5.0 mmol) were dissolved under argon with stirring in 24.0 ml ethyl acetate. The clear yellow solution was treated with 1.3 ml ethanol, 0.96 ml (5.0 mmol) dibenzylamine and 0.94 ml (5.5 mmol) N-ethyldiisopropylamine. The reaction mixture was heated to reflux for 2.5 h, then 0.05 ml (0.25 mmol) dibenzylamine and 0.04 ml (0.25 mmol) N-ethyldiisopropylamine were added and the mixture was refluxed for another 1.5 h. The clear yellow solution was cooled to room temperature and the solvent evaporated at 40°C and 200 to 5 mbar to yield 3.48 g crude product as a yellow oil. Purification of the crude product was obtained by column chromatography with 85 g silica using a mixture of dichloromethane, methanol, 25% aqueous ammonium hydroxide (90/10/1). The combined fractions were evaporated and dried on a rotary evaporator at 40°C and 250 to 10 mbar yielding 1.14 g 2,2-dibenzyl-4-fluoro-2,3-dihydro-1*H*-isoindolium methanesulfonate (55%) as a white solid.
MS (turbo spray) 318 (M⁺, 100), 319 (24), 226 (7)

### 3.4.c) Preparation of 4-fluoro-2,3-dihydro-1H-isoindol methanesulfonic acid (10)

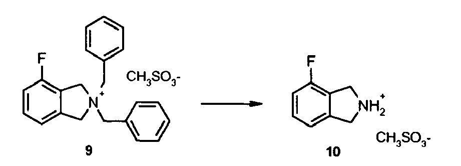

In a 10 ml two necked round bottom flask equipped with a thermometer a magnetic stirrer, and a hydrogen gas supply, 827 mg 2,2-dibenzyl-4-fluoro-2,3-dihydro-1*H*-isoindolium methanesulfonate (2.0 mmol) were dissolved in 4.1 ml methanol and 41.0 mg palladium on charcoal 10% was added. The vessel was evacuated three times and ventilated, first with argon, then with hydrogen. The black mixture was hydrogenated for 2.5 h at room temperature, filtered and the filter cake was washed with 6.0 ml methanol. The clear filtrate was evaporated at 40°C and 300 to 10 mbar yielding 451 mg (97%) of4-fluoro-2,3-dihydro-1*H*-isoindol methanesulfonic acid (10) as a grey solid.
MS (turbo spray) 138 (M+H⁺, 100), 139 (6)

### 4.1. Synthesis of 3-trifluoromethyl-6,7-dihydro-5H-pyrrolo[3,4-b]pyridin-6-ium chloride

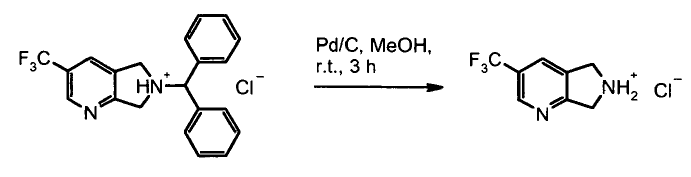

An autoclave was charged with a suspension of 150 g 6-benzhydryl-3-trifluoromethyl-6,7-dihydro-5H-pyrrolo[3,4-b] pyridine hydrochloride salt (383.8 mmol) and 7.5 g palladium on charcoal in 0.5 1 methanol. The suspension was stirred at room temperature. The autoclave was sealed, the hydrogen pressure set at 1.5 bar (absolute) and the hydrogenation was conducted under stirring at 20 - 25°C for 3 - 4 h. Then the autoclave was vented, the suspension filtered and the black residue was rinsed twice with 0.5 1, in total with 1 1 methanol. The solvent of the filtrate was evaporated at 60°C under reduced pressure to yield 152.3 g of crude 3-trifluoromethyl-6,7-dihydro-5H-pyrrolo[3,4-b]pyridin-6-ium chloride as brown residue.

The clear yellow solution of 152.3 g crude product 3-trifluoromethyl-6,7-dihydro-5H-pyrrolo[3,4-b]pyridin-6-ium chloride in 200 ml methanol was diluted with 600 ml ethyl acetate and the solvent was evaporated at 60°C under reduced pressure (335 - 250 mbar) to a total volume of 500 ml. Methanol was exchanged at 60°C, keeping the volume constant under reduced pressure by the addition of 3.5 1 ethyl acetate. The suspension formed was treated at 60°C with 125 ml toluene, stirred for 15 min and 50 ml methanol was added. After stirring for 15 min at 60°C the suspension was cooled over 1 h to room temperature and stirred for 1 h. The crystals were filtered and rinsed with a mixture of 300 ml ethyl acetate, 10 ml toluene and 10 ml methanol then dried at 60°C and <10 mbar for 2 h and at room temperature over night yielding 74.8 g (86.7%) of 3-trifluoromethyl-6,7-dihydro-5H-pyrrolo[3,4-b]pyridin-6-ium chloride as a beige powder.
MS (EI): 189 (M+H⁺, 100)

### 4.2. Synthesis of 3-trifluoromethyl-6,7-dihydro-5H-pyrrolo[3,4-b]pyridin-6-ium methanesufonate

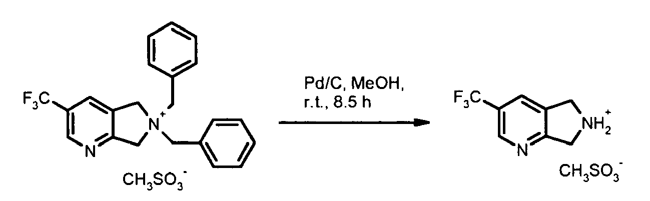

A 200 ml four-necked round bottom flask equipped with a thermometer probe dipping into reaction mixture, a mechanical hydrogenation stirrer, an inert gas supply and a hydrogenation supply apparatus was charged with a solution of 15.8 g 6,6-dibenzyl-3-trifluoromethyl-6,7-dihydro-5H-pyrrolo[3,4-b]pyridin-6-ium methanesulfonate (34.0 mmol) and 0.39 g palladium on charcoal in 79 ml methanol which was then stirred at room temperature. The reaction flask was ventilated several times with argon and hydrogen. The hydrogenation was performed under atmospheric pressure and stirred at 20 - 25°C for 8.5 h. Then the reaction flask was ventilated several times with argon and the suspension was filtered. The black residue was rinsed twice with 13 ml, in total with 26 ml methanol.

From the clear light brown filtrate, 80 ml methanol were distilled off (80°C/480 - 280 mbar), then the remaining methanol was exchanged at T 38 - 43°C and 560 - 370 mbar, keeping the volume constant by the addition oftwice 76 ml, in total 152 ml ethyl acetate. Crystallization commenced and a further 76 ml ethyl acetate were added. The light brown suspension was heated to reflux for 5 min, cooled to room temperature within 0.5 h and stirred for 1 h at room temperature. The suspension was filtered and the off-white crystals were rinsed in portions with a total of 25 ml ethyl acetate. The crystals were dried in a rotary evaporator at 42°C and <10 mbar for 6 h, yielding 8.87 g (91.8%) 3-trifluoromethyl-6,7-dihydro-5H-pyrrolo[3,4-b]pyridin-6-ium methanesufonate as an off-white powder
MS (EI): 189 (M+H⁺, 100).

### 5. Synthesis of (S)-trifluoro-isopropanol as described in WO 2008/012240.

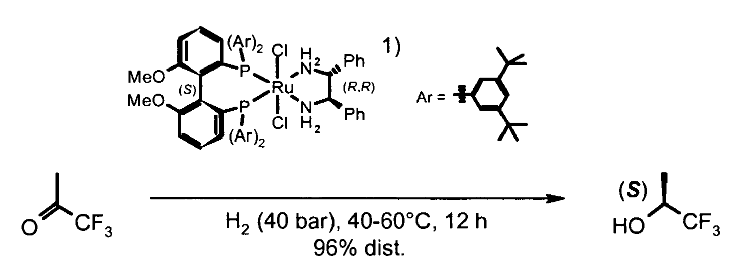

### 1) RuCl₂((S)-3,5-tBu-MeOBIPHEP)((R,R)-DPEN

The reaction was carried out in a 185-ml stirred autoclave equipped with an electric heating/cooling system, temperature cascade control, measurement of temperature, of gas flow, of stirring rate and of pressure. The whole system is connected to a hydrogen feed system. During the operation the temperature, the stirring rate, the hydrogen flow rate as well as the hydrogen consumption were recorded on-line through a PC data gathering system.

In the glove box the 185-ml autoclave was charged with 0.75 ml water and stored in the refrigerator (-18°C) for 2 h. Then, the autoclave was removed from the refrigerator and charged with 14.86 mg RuCl₂((S)-3,5-tBu-MeOBIPHEP)((R,R)-DPEN (10.5 x 10⁻³ mmol, substrate-to-catalyst ratio of 20'000), 75.00 mg sodium formate (1.10 mmol) and 23.44 g cold (-18°C) trifluoroacetone (18.75 ml, 209.2 mmol), sealed immediately and pressurized with 7 bar of argon.

The autoclave was removed from the glove box and introduced into a heating/cooling unit and connected to a hydrogenation line which was thoroughly flushed with hydrogen. The autoclave was pressurized with 40 bar of hydrogen under stirring and the heating started. After 10 h, the reaction temperature was increased to 60°C for 2 h to complete the conversion. Then the autoclave was cooled and vented and the crude reaction mixture transferred into a 30 ml roundbottomed flask to yield the crude 24.55 g of (S)-trifluoro-isopropanol as an off-white suspension Distillation: 24.55 g raw product were distilled at 34°C/150 mbar to yield 23.91 g (96.3%) of (*S*)-trifluoro-isopropanol as a colorless oil

| | |
|---|---|
| H₂O content | 3.4%; cf. EA |
| Ru-content | <1 ppm, not detected |

### 6. Synthesis of 5-methanesulfonyl-2-(2,2,2-trifluoro-1-methyl-ethoxy)-benzoic acid as described in EP application no. 07103485.4

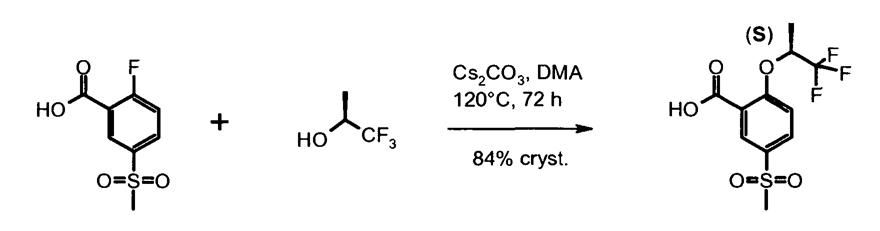

A 12 1 autoclave was charged with a colorless solution of 700.0 g 2-fluoro-5-methanesulfonyl-benzoic acid (3.2 mol) in 7.7 1 N,N-dimethyl-acetamide. The solution was treated with 1965.0 g cesium carbonate (6.0 mol) and 522.8 g (S)-trifluoro-isopropanol (4.6 mol). The white reaction suspension was warmed to 120°C and stirred under argon for 72 h (1.5 bar).

After cooling to 20°C, the white suspension was filtered, the filter cake was washed with 500 ml N,N-dimethyl-acetamide and the filtrate was evaporated. To the residue was added 9 1 water and the solution was extracted 3 times with 7 l, in total with 21 l ethyl acetate. The aqueous phase was heated in the rotary evaporator to completely remove residual ethyl acetate from the water phase. The pH of the water phase was adjusted to 1.5 by addition of 600 ml HCl 37%, whereby the product precipitated. The suspension was stirred at room temperature for 1 h, filtered, the crystals were washed with 5 1 water and dried under high vacuum for 24 h at 50°C to yield 840.0 g (84.0%) of 5-methanesulfonyl-2-(2,2,2-trifluoro-1-methyl-ethoxy)-benzoic acid as white crystals.

### 7.1. Synthesis of [5-methanesulfonyl-2-(2,2,2-trifluoro-1-methyl-ethoxy)-phenyl]-(3-trifluoromethyl-6,7-dihydro-5H-[1]pyridin-6-yl)-methanone

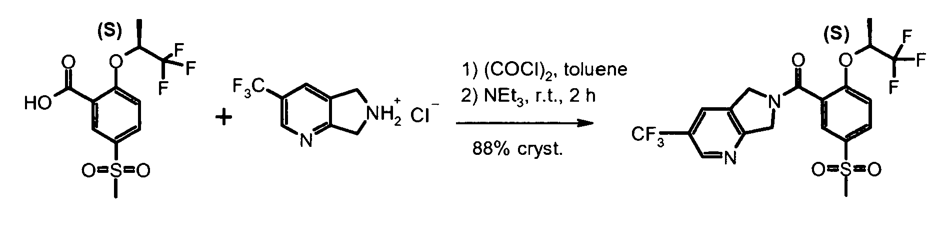

250 ml four-necked round bottom flask equipped with a thermometer, a dropping funnel, a mechanical stirrer and an inert gas supply

A beige suspension of 44.9 g 5-methanesulfonyl-2-(2,2,2-trifluoro-1-methyl-ethoxy)-benzoic acid (143.7 mmol) and 1.8 ml dimethylformamide in 295 ml toluene was treated at room temperature over 14 min with a solution of 18.3 g oxalyl chloride (141.3 mmol) in 32 ml toluene (exothermic reaction). The suspension was stirred for 1 h at room temperature and added dropwise at room temperature over 16 min into a 2.5 l four-necked round bottom flask charged with a solution of 32.0 g 1-(3-fluoro-5-trifluoromethyl-pyridin-2-yl)-piperazine HCl (142.5 mmol) of example 4.1. in 900 ml toluene and 61.3 g triethylamine (605.6 mmol). The reaction mixture was stirred at room temperature for 2.5 h.

The suspension was filtered and the residue washed in portions with 190 ml toluene. The filtrate was extracted with 860 ml water. After separation of the phases, the organic phase was washed three times with 590 ml, in total with 1.8 l sodium bicarbonate 5% and once with 600 ml NaCl solution 5%. The organic phase was dried over Na₂SO₄ filtrated and the solvent of the organic phase was evaporated at 60°C and 400 mbar. The residue was treated with 400 ml ethanol and the solvent again evaporated at 60°C under reduced pressure to yield crude 70.7 g of [5-methanesulfonyl-2-(2,2,2-trifluoro-1-methyl-ethoxy)-phenyl]-(3-trifluoromethyl-6,7-dihydro-5H-[1]pyridin-6-yl)-methanone as a light yellow foam.

Crystallization from ethanol/water: A yellow solution of 70.7 g [5-methanesulfonyl-2-(2,2,2-trifluoro-1-methyl-ethoxy)-phenyl]-(3-trifluoromethyl-6,7-dihydro-5H-[1]pyridin-6-yl)-methanone in 194 ml ethanol was heated under reflux and treated over 30 min with 194 ml water. The mixture was cooled over 1 h to T = 38 - 42°C and stirred for 1 h at this temperature (seeding at T = 53 - 55°C). To the white thick suspension was added within 47 min 580 ml water and the suspension was cooled within 30 min to room temperature and stirred for 1 h. The white suspension was filtered, the crystals were rinsed with a mixture of 32 ml ethanol and 128 ml water, then dried at 50°C and 5 mbar for 15 h to yield 62.7 g of [5-methanesulfonyl-2-(2,2,2-trifluoro-1-methyl-ethoxy)-phenyl]-(3-trifluoromethyl-6,7-dihydro-5H-[1]pyridin-6-yl)-methanone as a light yellow powder.

Crystallization from ethanol/heptane: A yellow solution of 62.7 g [5-methanesulfonyl-2-(2,2,2-trifluoro-1-methyl-ethoxy)-phenyl]-(3-trifluoromethyl-6,7-dihydro-5H-[1]pyridin-6-yl)-methanone in 150 ml ethanol was heated under reflux, stirred for 10 min, then cooled over 1 h to T = 38 - 42°C and stirred for 1 h at this temperature (seeding with form A at T = 53 - 55°C). The thick suspension was cooled over 60 min to room temperature and treated over 60 min with 450 ml heptane. After stirring for 1 h, the white suspension was filtered and the crystals were rinsed with a mixture of 34 ml ethanol and 66 ml heptane then dried at 50°C and <10 mbar over night to yield 58.8 g (88.0%) of [5-methanesulfonyl-2-(2,2,2-trifluoro-1-methyl-ethoxy)-phenyl]-(3-trifluoromethyl-6,7-dihydro-5H-[1]pyridin-6-yl)-methanone as white crystals^{*)}.
MS (EI): 505 (M+Na⁺, 44), 483 (M+H⁺, 100).
m.p.: 147.3°C

### 7.2. Synthesis of [5-methanesulfonyl-2-(2,2,2-trifluoro-1-methyl-ethoxy)-phenyl]-(3-trifluoromethyl-6,7-dihydro-5H-[1]pyridin-6-yl)-methanone

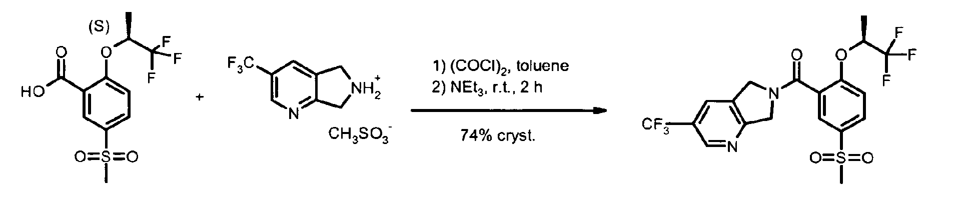

A 100 ml four-necked round bottom flask equipped with a thermometer, a dropping funnel, a mechanical stirrer and an inert gas supply was charged with an off-white suspension of 3.12 g 5-methanesulfonyl-2-(2,2,2-trifluoro-1-methyl-ethoxy)-benzoic acid (10.0 mmol) and 0.12 ml dimethylformamide in 39.0 ml toluene. The suspension was treated at room temperature over 10 min with a solution of 0.86 ml oxalyl chloride (10.0 mmol) in 2.2 ml toluene (exothermic reaction, gas evolution). The turbid solution was stirred for 1.5 h at room temperature and added at room temperature within 15 min into a 250 ml four-necked round bottom flask equipped as mentioned above charged with a solution of 2.84 g 3-trifluoromethyl-6,7-dihydro-5H-pyrrolo[3,4-b]pyridin-6-ium methanesulfonate (10.0 mmol) from example 4.2. in 84.0 ml ethyl acetate and 5.85 ml triethylamine (42.0 mmol). The reaction mixture was stirred at room temperature for 2.5 h.

The light brown suspension was extracted with 80.0 ml 1M aqueous hydrochloric acid. The organic phase was washed three times with 50 ml, in total with 150 ml sodium bicarbonate 1M and once with 50 ml NaCl solution 12%. The combined organic phase was dried over I 10 g Na₂SO₄, filtered over a glass fiber filter and the residue was washed in portions with total 60 ml ethyl acetate. The combined filtrates were evaporated (42°C and 400 - 5 mbar) and the residual off-white foam was treated with 20 ml ethanol. The solvent was removed at 42°C and 150 - 5 mbar yielding crude 4.47 g of [5-methanesulfonyl-2-(2,2,2-trifluoro-1-methyl-ethoxy)-phenyl]-(3-trifluoromethyl-6,7-dihydro-5H-[1]pyridin-6-yl)-methanone as off-white foam

Crystallization from ethanol/water: A yellow-brown solution of 4.45 g crude product in 13.4 ml ethanol was heated to 61°C and treated over 5 min with 13.4 ml water. The mixture was cooled over 1 h to T = 38 - 42°C and stirred for 1 h at this temperature. 39.5 ml water was added to the white thick suspension over 105 min and the suspension was cooled over 60 min to room temperature and stirred for 16 h. The white suspension was filtered, the crystals were rinsed with a mixture of 1.9 ml ethanol and 7.6 ml water and dried at 40°C and <10 mbar for 6 h yielding 4.03 g of [5-methanesulfonyl-2-(2,2,2-trifluoro-1-methyl-ethoxy)-phenyl]-(3-trifluoromethyl-6,7-dihydro-5H-[1]pyridin-6-yl)-methanone as a off-white powder

Crystallization from ethanol/heptane: A yellow-brown solution of 4.03 g [5-methanesulfonyl-2-(2,2,2-trifluoro-1-methyl-ethoxy)-phenyl]-(3-trifluoromethyl-6,7-dihydro-5H-[1]pyridin-6-yl)-methanone in 9.3 ml ethanol was heated under reflux, stirred for 3 min, cooled over 35 min to T = 38 - 42°C and stirred for 2.5 h at this temperature whereby an off-white suspension was formed. This suspension was cooled over 60 min to room temperature, stirred for 16 h, treated within 16 min dropwise with 27 ml heptane and stirred at room temperature again for 6 h. The white suspension was filtered, the crystals were washed with a mixture of 1.5 ml Ethanol and 4.5 ml heptane then dried over night at 40°C and <10 mbar yielding 3.55 g (74%) of [5-methanesulfonyl-2-(2,2,2-trifluoro-1-methyl-ethoxy)-phenyl]-(3-trifluoromethyl-6,7-dihydro-5H-[1]pyridin-6-yl)-methanone as white crystals^{*)}
MS (EI): 505 (M+Na⁺, 32), 483 (M+H⁺, 100).

## Claims

1. A process for the manufacture of a compound of formula II or a salt thereof 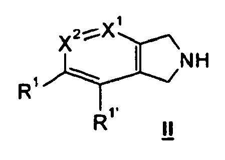 wherein
X¹ is N or C;
X² is C, or when X¹ is C, X² is C or N;
R¹ and R^{1'} are independently of each other hydrogen, hydroxy, halogen, C₁-C₆ alkyl optionally substituted by halogen or hydroxy, lower alkoxy optionally substituted by halogen, cycloalkyl, -CN, -NH₂, -S-C₁-C₆ alkyl, -S(O)₂-C₁-C₆ alkyl, -O-(CH₂)_{y}-C₁-C₆ alkoxy, -O(CH₂)_{y}C(O)N(C₁-C₆ alkyl)₂, -C(O)-C₁-C₆ alkyl, -C(O)O-C₁-C₆ alkyl, -C(O)-NH-C₁-C₆ alkyl, or -C(O)-N(C₁-C₆ alkyl)₂.
which process comprises
a) carbonylation of a compound of formula **1** 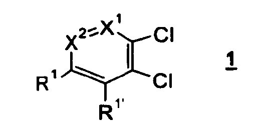 to obtain the diester of formula **2** 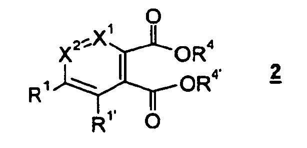 wherein R⁴, and R^{4'} are idependently C₁-C₆-alkyl;
in the presence of CO and a catalysator;
b) reduction of the diester **2** to the diol **3** 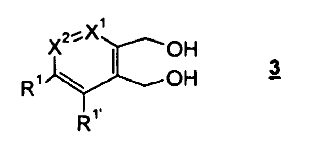 in the presence of reduction reagent selected from the group NaBH₄ in acetic acid, (diisobutyl aluminum hydride), LiBH₄ or LiAlH₄ and optionally in the presence of an alkali and/or alkaline earth salt or transition metal salt
c) formation of the diol derivative **4** 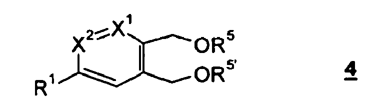 wherein
R⁵ and R^{5'} are independently a leaving group selected from nosylate, tosylate, mesylate;
in presence of a base, in a solvent wherein the salts formed during the reaction are insoluble and easily removeable by filtration;
d) cyclisation of the compound **4** with an amino derivative HNR⁶R^{6'} (**5**) 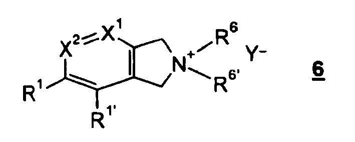 wherein
R⁶, R^{6,}, are independently of each other hydrogen or an amino protecting group; and
Y⁻ is an anion of a corresponding salt;
in the presence of a base; or, if one of R⁶, R^{6'} is an amino protecting group,
e) hydrogenolysis of the protected amine 6 or its salt to the amine of formula II or a salt thereof: 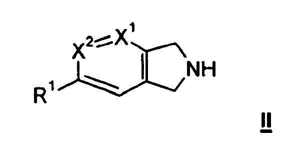 with hydrogen in the presence of catalytic amounts of Pd/C, optionally in the presence of an acid to yield the corresponding salt of formula II.

2. A process for the manufacture of a compound of formula II or a salt thereof as defined in claim 1, which process comprises
cyclisation of the compound **4** 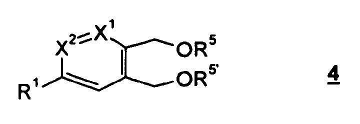 wherein
R¹, R^{1'}, X¹ and X² are as defined in claim 1,
R⁵ and R^{5'} are independently a leaving group selected from nosylate, tosylate, mesylate;
with an amino derivative HNR⁶R^{6'} (**5**) 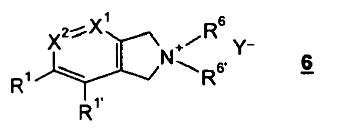 wherein
R¹, R^{1'}, X¹ and X² are as defined in claim 1,
R⁶, R^{6',}, are independently of each other hydrogen or an amino protecting group; and
Y⁻ is an anion of a corresponding salt;
in the presence of a base; and subsequent hydrogenolysis of the protected amine 6, if one of R⁶, R^{6'} is an amino protecting group, or its salt to the amine of formula II or a salt thereof: 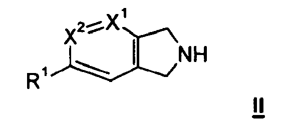 wherein R¹, X¹ and X² are defined in claim 1,
with hydrogen in the presence of catalytic amounts of Pd/C, optionally in the presence of an acid to yield the corresponding salt of formula II.

## Patentansprüche

1. Verfahren zur Herstellung einer Verbindung der Formel II oder eines Salzes davon: 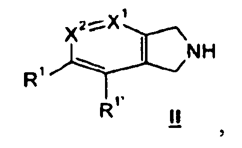 wobei:
X¹ N oder C ist;
X² C ist, oder, wenn X¹ C ist, X² C oder N ist;
R¹ und R^{1'} unabhängig voneinander Wasserstoff, Hydroxy, Halogen, C₁-C₆-Alkyl, gegebenenfalls substituiert durch Halogen oder Hydroxy, Niederalkoxy, gegebenenfalls substituiert durch Halogen, Cycloalkyl, -CN, -NH₂, -S-C₁-C₆-Alkyl, -S(O2-C₁-C₆-Alkyl, -O-(CH₂)_{y}-C₁-C₆-Alkoxy, -O(CH₂)_{y}C(O)N(C₁-C₆-Alkyl)₂, -C(O)-C₁-C₆-Alkyl, -C(O)O-C₁-C₆-Alkyl, -C(O)-NH-C₁-C₆-Alkyl oder -C(O)-N(C₁-C₆-Alkyl)₂ sind;
wobei das Verfahren
a) Carbonylierung einer Verbindung der Formel 1 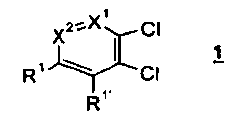 unter Erhalt des Diesters der Formel 2 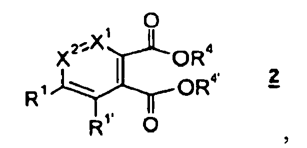 wobei R⁴ und R^{4'} unabhängig C₁-C₆-Alkyl sind;
in Gegenwart von CO und eines Katalysators;
b) Reduktion des Diesters 2 zu dem Diol 3 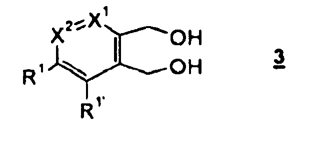 in Gegenwart eines Reduktionsreagens, ausgewählt aus der Gruppe NaBH₄ in Essigsäure, (Diisobutylaluminiumhydrid), LiBH₄ oder LiAlH₄, und gegebenenfalls in Gegenwart eines Alkalimetall- und/oder Erdalkalimetallsalzes oder Übergangsmetallsalzes;
c) Bildung des Diolderivats 4 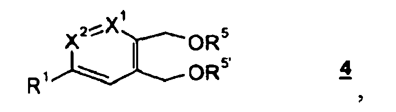 wobei
R⁵ und R^{5'} unabhängig eine Austrittsgruppe, ausgewählt aus Nosylat, Tosylat oder Mesylat, sind;
in Gegenwart einer Base in einem Lösungsmittel, wobei die während der Reaktion gebildeten Salze unlöslich und durch Filtration leicht zu entfernen sind;
d) Cyclisierung der Verbindung 4 mit einem Aminoderivat HNR⁶R^{6'} (5) 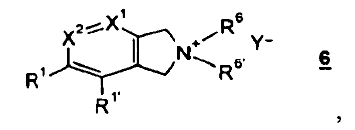 wobei
R⁶, R^{6'} unabhängig voneinander Wasserstoff oder eine Aminoschutzgruppe sind und
Y⁻ ein Anion eines entsprechenden Salzes ist;
in Gegenwart einer Base; oder, wenn einer von R⁶, R^{6'} eine Aminoschutzgruppe ist,
e) Hydrogenolyse des geschützten Amins 6 oder seines Salzes zu dem Amin der Formel II oder einem Salz davon: 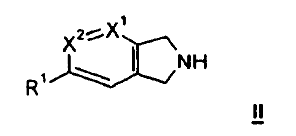 mit Wasserstoff in Gegenwart katalytischer Mengen Pd/C, gegebenenfalls in Gegenwart einer Säure, unter Erhalt des entsprechenden Salzes der Formel II umfasst.

2. Verfahren zur Herstellung einer Verbindung der Formel II oder eines Salzes davon, wie in Anspruch 1 definiert, wobei das Verfahren
Cyclisierung der Verbindung 4 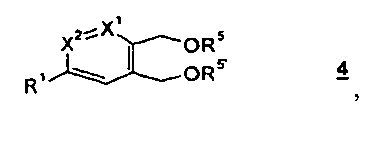 wobei
R¹, R^{1'}, X¹ und X² wie in Anspruch 1 definiert sind,
R⁵ und R^{5'} unabhängig eine Austrittsgruppe, ausgewählt aus Nosylat, Tosylat, Mesylat, sind; mit einem Aminoderivat HNR⁶R^{6'} (5) 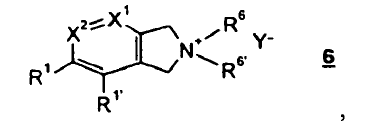
wobei
R¹, R^{1'}, X¹ und X² wie in Anspruch 1 definiert sind,
R⁶, R^{6'} unabhängig voneinander Wasserstoff oder eine Aminoschutzgruppe sind und
Y⁻ ein Anion eines entsprechenden Salzes ist;
in Gegenwart einer Base und anschließende Hydrogenolyse des geschützten Amins 6, wenn einer von R⁶, R^{6'} eine Aminoschutzgruppe ist, oder seines Salzes zu dem Amin der Formel II oder einem Salz davon: 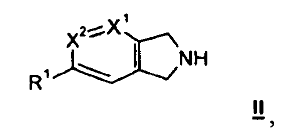 wobei R¹, X¹ und X² wie in Anspruch 1 definiert sind,
mit Wasserstoff in Gegenwart katalytischer Mengen Pd/C, gegebenenfalls in Gegenwart einer Säure, unter Erhalt des entsprechenden Salzes der Formel II umfasst.

## Revendications

1. Procédé de préparation d'un composé de formule II ou d'un de ses sels 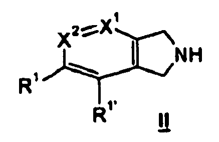 où
X¹ est N ou C;
X² est C, ou, lorsque X¹ est C, X² est C ou N;
R¹ et R^{1'} sont, indépendamment l'un de l'autre, hydrogène, hydroxy, halogène, alkyle en C₁-C₆ éventuellement substitué par halogène ou hydroxy, alcoxy inférieur éventuellement substitué par halogène, cycloalkyle, -CN, -NH₂, -S-alkyle en C₁-C₆, -S(O)₂-alkyle en C₁-C₆, -O-(CH₂)_{y}-(alcoxy en C₁-C₆), -O-(CH₂)_{y}-C(O)N(alkyle en C₁-C₆)₂, -C(O)-(alkyle en C₁-C₆), -C(O)O-(alkyle en C₁-C₆), -C(O)-NH-(alkyle en C₁-C₆) ou -C(O)-N(alkyle en C₁-C₆)₂,
ce procédé comprenant:
a) la carbonylation d'un composé de formule **1** 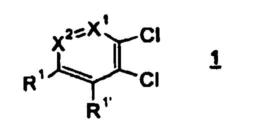 pour l'obtention du diester de formule **2** 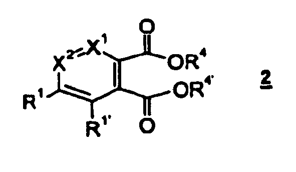 où R⁴ et R^{4'} sont indépendamment alkyle en C₁-C₆;
en présence de CO et d'un catalyseur;
b) la réduction du diester **2** en le diol **3** 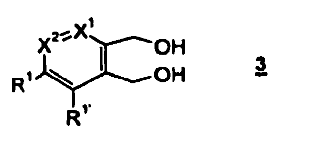 en présence d'un réactif de réduction choisi dans le groupe NaBH₄ dans de l'acide acétique, hydrure de diisobutylaluminium, LiBH₄ ou LiAlH₄ et éventuellement en présence d'un sel de métal alcalin et/ou de métal alcalino-terreux ou d'un sel de métal de transition;
c) la formation du dérivé de diol **4** 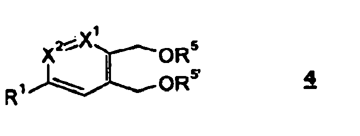 où R⁵ et R^{5'} sont indépendamment un groupe partant choisi parmi un nosylate, un tosylate, un mésylate;
en présence d'une base, dans un solvant dans lequel les sels formés pendant la réaction sont insolubles et faciles à séparer par filtration;
d) la cyclisation du composé **4** avec un dérivé amino HNR⁶R^{6'} (**5**) 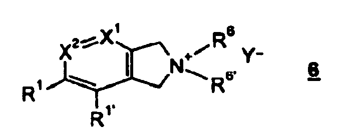 où
R⁶ et R^{6'} sont, indépendamment l'un de l'autre, un hydrogène ou un groupe protecteur d'amino; et
Y⁻ est un anion d'un sel correspondant;
en présence d'une base; ou, si l'un des R⁶ et R^{6'} est un groupe protecteur d'amino,
e) l'hydrogénolyse de l'amine protégée **6** ou de son sel en l'amine de formule II ou un de ses sels: 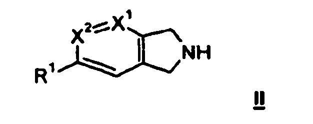 avec de l'hydrogène en présence de quantités catalytiques de Pd/C, éventuellement en présence d'un acide pour l'obtention du sel correspondant de formule II.

2. Procédé de préparation d'un composé de formule II ou d'un de ses sels tels que définis dans la revendication 1, ce procédé comprenant la cyclisation du composé **4** 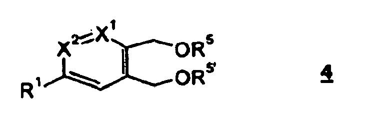 où
R¹, R^{1'}, X¹ et X² sont tels que définis dans la revendication 1,
R⁵ et R^{5'} sont indépendamment un groupe partant choisi parmi un nosylate, un tosylate, un mésylate;
avec un dérivé amino HNR⁶R^{6'} (5) 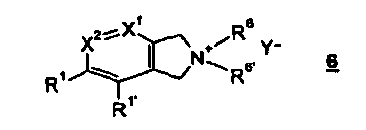 où
R¹, R^{1'}, X¹ et X² sont tels que définis dans la revendication 1,
R⁶ et R^{6'} sont, indépendamment l'un de l'autre, un hydrogène ou un groupe protecteur d'amino; et
Y⁻ est un anion d'un sel correspondant;
en présence d'une base; puis l'hydrogénolyse de l'amine protégée **6**, si l'un des R⁶ et R^{6'} est un groupe protecteur d'amino, ou de son sel, en l'amine de formule II ou un de ses sels: 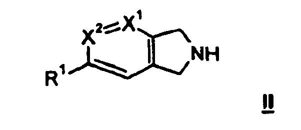 où R¹, X¹ et X² sont définis dans la revendication 1,
avec de l'hydrogène en présence de quantités catalytiques de Pd/C, éventuellement en présence d'un acide pour l'obtention du sel correspondant de formule II.
